# EUROPEAN PATENT APPLICATION

(11) **EP 4 338 660 A1**
(43) Date of publication of application: **20.03.2024**
(21) Application number: 22196148.5
(22) Date of filing: 16.09.2022
(51) Int. Cl.: A61B 5/00

(54) **METHOD AND SYSTEM FOR PROVIDING BASIS FOR A WEIGHT CHANGE INVARIANT BODY COMPOSITION EVALUATION**

(71) Applicant: AMRA Medical AB, 582 22 Linköping (SE)
(72) Inventor: Linge, Jennifer, 582 24 Linköping (SE)
(74) Representative: AWA Sweden AB

(57) **Abstract**

The present invention relates to a system and a method of providing a basis for a weight change invariant body composition evaluation for a subject individual is provided. A first and a second value of a body parameter of the subject individual are acquired for a first and a second point in time respectively. A first and a second value of a subject characteristic parameter comprising weight of the subject individual are acquired for the first and the second points in time. Two groups of individuals are selected from databases based on comparison with the subject characteristic parameter. Mean or median values and optionally standard deviations of the distribution of the body parameter values for the selected individuals in the groups of individuals are determined. The basis for the evaluation is provided by a determined first body parameter change based on the mean or median values in combination with the first and second values of the body parameter of the subject individual.

## Description

### Technical Field

The present disclosure relates to a method and a system of providing a basis for weight change invariant body composition evaluation for a subject individual.

### Background

In many treatment situations, weight loss or weight gain may be studied to evaluate the treatment. However, only evaluating the weight loss or gain may not provide a correct view of the development for the individual. There are both good and bad weight loss or weight gain, e.g. provided by a differentiation between different weight loss treatments, such as preserving muscle health or shifting fat distribution. Hence, there is a need for a basis for such evaluation, that takes into account further aspects of the body composition of the individual.

### Summary

It is an object of the present invention to provide an improved solution that alleviates the mentioned drawbacks with present methods. Furthermore, it is an object to provide a method and system for providing a basis for a weight change invariant body composition evaluation for a subject individual.

The invention is defined by the appended independent claims, with embodiments being set forth in the appended dependent claims, in the following description and in the drawings.

According a first aspect of the invention, a method of providing a basis for a weight change invariant body composition evaluation for a subject individual is provided. The method is performed by a processing unit and comprising the steps of: acquiring a first value of a body parameter of the subject individual for a first point in time, the body parameter being a fat or muscle related parameter; acquiring a second value of the body parameter of the subject individual for a second point in time being different from the first point in time; acquiring a first value of a first subject characteristic parameter of the subject individual for the first point in time, the first subject characteristic parameter comprising weight optionally in combination with one or more of height, age, sex, ethnicity, BMI or other body measurement; acquiring a second value of a second subject characteristic parameter of the subject individual for the second point in time, the second subject characteristic parameter comprising weight optionally in combination with one or more of height, age, sex, ethnicity and BMI; selecting a first group of individuals from a first database, wherein the first database comprises, for each of a plurality of individuals, values of the body parameter and the first subject characteristic parameter, wherein the selection of the first group of individuals from the first database is based on the first subject characteristic parameter values for the selected individuals being similar to the first value of the first subject characteristic parameter for the subject individual; selecting a second group of individuals from a second database, wherein the second database comprises, for each of the plurality of individuals, values of the body parameter and the second subject characteristic parameter, wherein the selection of the second group of individuals from the second database is based on the second subject characteristic parameter values of the selected individuals being similar to the second value of the second subject characteristic parameter for the subject individual; determining a first mean or median value of the distribution of the body parameter values for the individuals in the first group of individuals; determining a second mean or median value of the distribution of the body parameter values for the individuals in the second group of individuals; determining a first body parameter change by comparing the first mean or median value, optionally in combination with a first standard deviation of the distribution of the body parameter values for the individuals in the first group of individuals, with the second mean or median value, optionally in combination with a second standard deviation of the distribution of the body parameter values for the individuals in the second group of individuals, in combination with the first value and the second value of the body parameter of the subject individual.

The determined first body parameter change may thereby provide a basis for an effective evaluation of the body composition for a subject individual, that is weight change invariant. Since the body parameter values for the two different points in time use different groups of individuals from the databases as reference, the weight change itself for the subject individual may not affect the evaluation. Instead, the two body parameter values may be evaluated using a reference group that is relevant for the value of the body parameter at the particular time. The body parameters may correlate with the weight of the subject individual. The body parameter may indicate how much weight the subject individual should lose or gain. The body parameter value at the second point in time being compared to values based on a parameter comprising weight for a reference group relevant for the subject individual's weight at that time may thereby make the body parameter change determination more relevant for the evaluation.

By weight change invariant body composition evaluation it may be meant that evaluation is based on a determination that takes the weight change between different points in time into account. Between the two points in time, the first and second values of the body parameter may have changed (increased or decreased) by an absolute value. This absolute value may not provide a completely true picture for an evaluation of the subject individual's body composition. By using the method of the present invention, wherein reference groups are used which make the body parameter change weight invariant, a truer picture of the change in the body parameter values between the two points in time is provided. The body composition evaluation may then be performed with an improved basis.

The body parameter may be a measurable parameter of the body of the subject individual, defining a parameter of the composition of the body. The body parameter is fat or muscle related. By fat or muscle related it may be meant a parameter of one or more fat or muscle compartments in the body, such as volume or weight of such compartment, or a parameter being based on one or more fat or muscle compartment, such as a parameter being based on a volume or weight of such compartment.

The first point in time is different from the second point in time. The second point in time may be a later point in time than the first point in time.

The first subject characteristic parameter is a parameter comprising weight of the subject individual. Optionally, the first subject characteristic parameter comprises a combination between weight of the subject individual and one or more of height, age, sex, ethnicity, BMI or another body measurement of the subject individual. The other body measurement may be another measurable entity of the subject individual. The same applies to the second subject characteristic parameter.

The first database may comprise data of a large number of individuals. For each individual in the first database, values of at least the first subject characteristic parameter and the body parameter are stored. The individuals for the first group may be selected based on the first subject characteristic parameter for each selected individual being similar, e.g. within a predetermined interval, to the first value of the first characteristic parameter of the subject individual. When the first characteristic parameter comprises a combination of weight and e.g. age, the selection may be made in two steps, wherein a first selection may be made based on weight. Among the individuals selected in the first selection, the first group of individuals may be selected based on age. If the first subject characteristic parameter is a combination of more than two entities (such as weight, age and BMI), The selection of the first group may be made in three or more steps in the same way. For each entity the selection of the first group is based on (weight, height, age, sex, ethnicity, BMI, other body measurement), a predetermined interval may be set within which the entity value should be within from the value of the subject individual to be determined as similar to the value of the subject individual. The interval for an entity used for the selection to determine a selected individual to have e.g. weight similar to the subject individual may in one embodiment be adapted to the number of individuals in the database having the subject characteristic parameter within the interval. The number of individuals to be selected for the first group may be predetermined. The same applies to the second subject characteristic parameter and the selection of the second group of individuals from the second database. The number of individuals selected for the two groups may be the same.

For each individual in the selected first group of individuals, the first database comprises a value of the body parameter. If the first group comprises N selected individuals, N number of values of the body parameter may be determined. The first mean or median value may be based on the N number of values of the body parameter. Further, if determining a first standard deviation of the distribution of the body parameter values, the first standard deviation may be based on the N number of values of the body parameter, for the N number of individuals in the first group. The same applies to the second mean or median value and the optional second standard deviation. The second group may comprise M individuals, and the second mean or median value, and the second standard deviation, may be based on the M number values of the body parameter in the second database for the individuals in the second group.

The processing unit may comprise one or more computer units, data processing units, and/or general purpose processors configured to perform the steps of the method according to any of the embodiments herein. The processing unit may comprise a computer program or software configured to, when executed, to perform the steps of the method. The processing unit may comprise, or be communicatively connected to, a computer readable storage medium comprising the computer program or software.

In one embodiment, the method may further comprise the steps of determining a first standard deviation of the distribution of the body parameter values for the individuals in the first group of individuals, and a second standard deviation of the distribution of the body parameter values for the individuals in the second group of individuals; determining a first z-score by determining the number of first standard deviations of the first value of the body parameter from the first mean or median value; determining a second z-score by determining the number of second standard deviations of the second value of the body parameter from the second mean or median value; and the step of determining a first body parameter change may be performed by comparing the first z-score and the second z-score.

The first z-score may be determined based on the first value of the body parameter of the subject individual, the first mean or median value, and the first standard deviation. The difference between the first value of the body parameter of the subject individual and the first mean or median value may be determined in number of first standard deviations. That difference may provide the first z-score. The same applies to the second z-score being determined based on the second value of the body parameter, the second mean or median value and the second standard deviation.

In one embodiment, the method may further comprise the steps of determining a predicted change value by comparing the first mean or median value with the second mean or median value; determining a body parameter change value by comparing the first value of the body parameter with the second value of the body parameter; and the step of determining the first body parameter change may be performed by comparing the predicted change value and the body parameter change value.

The predicted change value may provide a difference between the first mean or median value and the second mean or median value. The body parameter change value may provide a difference between the first value and the second value of the body parameter of the subject individual. Based on these two differences being compared, a body parameter change may be determined. Hence, the deviation between the predicted body parameter change from the mean or median value for the reference groups, and the actual body parameter change for the subject individual may be determined. A deviation in an absolute value from the predicted change value based on the reference groups may thereby be provided. As an example, the subject individual may be determined to have lost 2 liters more visceral adipose tissue than the reference groups. This may provide a basis for the evaluation of the body parameter of the subject individual.

In one embodiment, the method may further comprise a step of combining the determined first body parameter change based on the z-scores with the determined body parameter deviation value. A more complete basis for the evaluation may thereby be provided, also including the deviation in absolute value.

In one embodiment, the body parameter may be a parameter out of the group of amount of visceral adipose tissue, amount of abdominal subcutaneous adipose tissue, amount of liver fat, muscle volume, muscle fat infiltration, organ volume, and organ fat infiltration.

In one embodiment, the first and second points in time may be separated in time by at least one week, at least 1 month, at least 6 months, about 10-14 months, at least 12 months. The second point in time may thereby be at least one week, at least 1 month, at least 6 months, about 10-14 months, or at least 12 months later than the first point in time. The first and second points in time may be the points in time which the first and second values of the body parameter for the subject individual, and the values of first and second subject characteristic parameters for the subject individual, relate to. I.e. the first value of the body parameter may be the value of the body parameter for the subject individual at the first point in time. Correspondingly, the second value may be the value of the body parameter for the subject individual at the second point in time. The same applies to the values of the first and second subject characteristic parameters for the subject individual.

In one embodiment, the first subject characteristic parameter and the second subject characteristic parameter may be the same subject characteristic parameter. Hence, both the first and second characteristic parameters may be weight in optional combination with the same one or more other body measurement, such as height, age, sex, ethnicity, BMI.

In one embodiment, the first subject characteristic parameter and/or the second subject characteristic parameter may be weight for the subject individual in combination with the sex of the subject individual.

In one embodiment, the first subject characteristic parameter and/or the second subject characteristic parameter may be weight for the subject individual in combination with age of the subject individual.

In one embodiment, the first subject characteristic parameter and/or the second subject characteristic parameter may be weight for the subject individual in combination with height of the subject individual.

In one embodiment, the first subject characteristic parameter and/or the second subject characteristic parameter may be weight for the subject individual in combination with ethnicity of the subject individual.

In one embodiment, the first subject characteristic parameter and/or the second subject characteristic parameter may be weight for the subject individual in combination with BMI of the subject individual.

In one embodiment, the selection of the first and/or second group of individuals may be made by selecting individuals from the first and/or second database having the first and/or second subject characteristic parameter value within a predetermined interval of the first and/or second value of the first and/or second subject characteristic parameter of the subject individual. The predetermined interval may be based on the number of individuals desired for the first and/or second group, the absolute value of the first and/or second value of the subject characteristic parameters, or the number of individuals in the first and/or second database. The predetermined interval may be changed in an iterative process if a desired number of individuals in the first and/or second group of individuals has not been reached in a first iteration of the selection of individuals.

In one embodiment, the first database and the second database may be the same database comprising, for each of the plurality of individuals therein, values of the body parameter, the first subject characteristic parameter, and the second subject characteristic parameter.

In one embodiment, the step of determining the first body parameter change may comprise determining a difference between the first z-score and the second z-score or a difference between the predicted change value and the body parameter change value. The difference between the z-scores or the predicted change value and body parameter change value may provide an output as a number that may form the basis for the weight change invariant body composition evaluation.

In one embodiment, the first and second values of the body parameter of the subject individual may be acquired based on MRI. In one embodiment, the steps of acquiring one or more of the values of the body parameter and the values of the first and/or second subject characteristic parameters may comprise receiving said values from a measurement unit and/or a storage unit. In another embodiment, said steps may comprise performing measurements and/or determinations of said values. Such measurements and/or determinations may at least partly be based on MRI.

In one embodiment, the first group and/or the second group of individuals may comprise at least 10, at least 50 or at least 100 individuals selected from the first database and/or the second database.

In one embodiment, the body parameter may be a first body parameter, and the method may further comprise the steps of: acquiring a first value of a second body parameter of the subject individual for the first point in time, the second body parameter being a fat or muscle related parameter different from the first body parameter; acquiring a second value of the second body parameter of the subject individual for the second point in time; wherein the first database further comprises, for each of the plurality of individuals therein, values of the second body parameter; wherein the second database further comprises, for each of the plurality of individuals therein, values of the second body parameter; determining a third mean or median value and a third standard deviation of the distribution of the second body parameter for the individuals in the first group of individuals; determining a fourth mean or median value and a fourth standard deviation of the distribution of the second body parameter for the individuals in the second group of individuals; determining a third z-score by determining the number of third standard deviations of the first value of the second body parameter from the third mean or median value; determining a fourth z-score by determining the number of fourth standard deviations of the second value of the second body parameter from the fourth mean or median value; determining a second body parameter change by comparing the third z-score and the fourth z-score; and combining the first body parameter change and the second body parameter change.

By determining a second body parameter change, and combining it with the first body parameter change, a more complex basis for the body composition evaluation may be provided, which may not be achieved using a single body parameter change basis. Two different body parameters may be evaluated and compared with reference groups using the method of the present invention. Connections between the two body parameters may be determined and seen in the determined basis, thereby providing a basis for more complex evaluation.

In one embodiment, the second body parameter may be a parameter out of the group of amount of visceral adipose tissue, amount of abdominal subcutaneous adipose tissue, amount of liver fat, muscle volume, muscle fat infiltration, organ volume, and organ fat infiltration. The second body parameter is a parameter out of the group above different from the first body parameter.

According to a second aspect of the present invention, a system for providing a basis for an evaluation of a body parameter distribution weight change for a subject individual is provided, the system comprising a processing unit configured to: acquire a first value of a body parameter of the subject individual for a first point in time, the body parameter being a fat or muscle related parameter; acquire a second value of the body parameter of the subject individual for a second point in time being different from the first point in time; acquire a first value of a first subject characteristic parameter of the subject individual for the first point in time, the first subject characteristic parameter comprising weight optionally in combination with one or more of height, age, sex, ethnicity, BMI or other body measurement; acquire a second value of a second subject characteristic parameter of the subject individual for the second point in time, the second subject characteristic parameter comprising weight optionally in combination with one or more of height, age, sex, ethnicity and BMI; select a first group of individuals from a first database, wherein the first database comprises, for each of a plurality of individuals, values of the body parameter and the first subject characteristic parameter, wherein the selection of the first group of individuals from the first database is based on the first subject characteristic parameter values for the selected individuals being similar to the first value of the first subject characteristic parameter for the subject individual; select a second group of individuals from a second database, wherein the second database comprises, for each of the plurality of individuals, values of the body parameter and the second subject characteristic parameter, wherein the selection of the second group of individuals from the second database is based on the second subject characteristic parameter values of the selected individuals being similar to the second value of the second subject characteristic parameter for the subject individual; determine a first mean or median value of the distribution of the body parameter values for the individuals in the first group of individuals; determine a second mean or median of the distribution of the body parameter values for the individuals in the second group of individuals; determining a first body parameter change by comparing the first mean or median value, optionally in combination with a first standard deviation of the distribution of the body parameter values for the individuals in the first group of individuals, with the second mean or median value, optionally in combination with a second standard deviation of the distribution of the body parameter values for the individuals in the second group of individuals, in combination with the first value and the second value of the body parameter of the subject individual.

The system may receive the first value of the body parameter measured or determined by another entity, such as by an acquisition unit, or the system may comprise an acquisition unit configured to measure or determine the first value. The measurement or determination may be based on an MRI scanning of the subject individual. The first value of the first subject characteristic parameter of the subject individual is acquired. Similarly as for the first value of the body parameter, the system may receive the first value of the subject characteristic parameter from the acquisition unit, or comprise the acquisition unit. The processing unit is configured to acquire the first values of the body parameter and the first subject characteristic parameter. The acquisition may be directly from the acquisition unit, or from a storage unit previously received the first values. The acquisition unit and/or storage unit may optionally be included in the system.

The processing unit may comprise one or more computer units, data processing units, general purpose processors or the like configured according to any of the embodiments herein. The processing unit may comprise a computer program or software configured to, when executed, to function according to any of the embodiments herein. The processing unit may comprise, or be communicatively connected to, a computer readable storage medium comprising the computer program or software.

In one embodiment, the processing unit may be configured to determine a first standard deviation of the distribution of the body parameter values for the individuals in the first group of individuals and a second standard deviation of the distribution of the body parameter values for the individuals in the second group of individuals; determine a first z-score by determining the number of first standard deviations of the first value of the body parameter from the first mean or median value; determine a second z-score by determining the number of second standard deviations of the second value of the body parameter from the second mean or median value; and wherein the determination of the first body parameter change may be performed by comparing the first z-score and the second z-score.

What is further discussed above for embodiments of the method according to the first aspect of the invention is equally applicable to embodiments of the system of the second aspect of the invention. The processing unit may be configured to perform such steps according to embodiments described above.

### Brief Description of the Drawings

The invention will in the following be described in more detail with reference to the enclosed drawings, wherein:
Fig. 1 shows a flow chart of a method according to an embodiment of the present invention.
Fig. 2 shows a block scheme of the method implemented by a system according to an embodiment of the present invention.
Fig. 3 shows a block scheme of the method implemented by a system according to an embodiment of the present invention.
Fig. 4 shows a block scheme of the method implemented by a system according to an embodiment of the present invention.
Fig. 5 shows a block scheme of a system according to an embodiment of the present invention.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

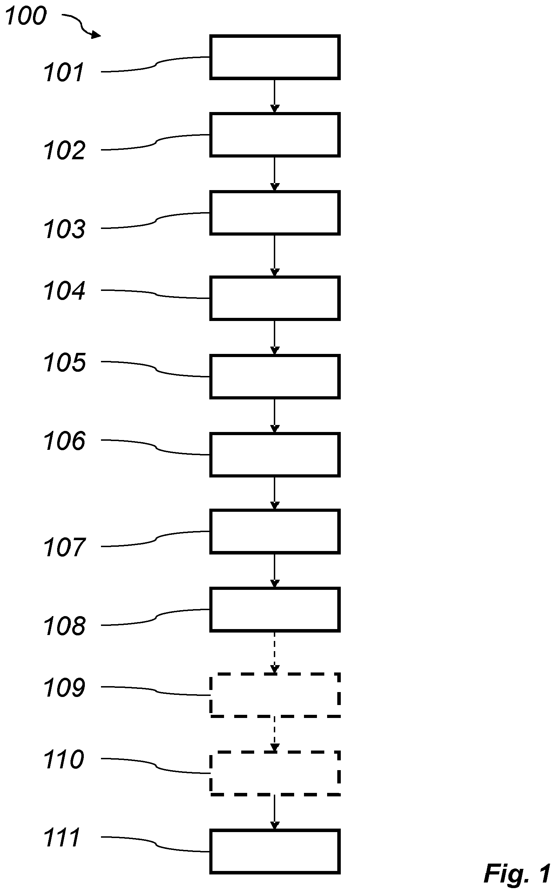

Fig. 1 illustrates a method 100 of providing a basis for a weight change invariant body composition evaluation for a subject individual 1. The method 100 comprises steps of acquiring two values of a first body parameter of the subject individual, the two values are acquired for the subject individual 1 at separate points in time T1, T2. At a first step of the method 100, a first value 10 of the first body parameter BP1 of the subject individual 1 for a first point in time T1 is acquired 101. The body parameter BP1 is a fat or muscle related parameter, for example amount of visceral adipose tissue, amount of abdominal subcutaneous adipose tissue, amount of liver fat, muscle volume, muscle fat infiltration, organ volume, and organ fat infiltration.

In a further step a second value 20 of the first body parameter BP1 of the subject individual 1 for a second point in time T2 is acquired 102. The second point in time T2 is different from the first point in time T1. The two points in time are preferably separated by at least one month, at least six months, between 10-14 months, or at least 12 months.

Besides the values of the first body parameter BP1, values of a first and second subject characteristic parameter SCP1, SCP2 of the subject individual 1 are acquired. A first value 11 of a first subject characteristic parameter SCP1 of the subject individual 1 for the first point in time T1 is acquired 103 and a second value 21 of a second subject characteristic parameter SCP2 of the subject individual for the second point in time T2 is acquired 104. The first subject characteristic parameter SCP1 comprises weight optionally in combination with one or more of height, age, sex, ethnicity, BMI or other body measurement. The second subject characteristic parameter SCP2 correspondingly comprises weight optionally in combination with one or more of height, age, sex, ethnicity and BMI. In some embodiments, the first and second subject characteristic parameters SCP1, SCP2 are the same, such as weight in combination with BMI, age and/or sex.

Further, the method 100 comprises a step of selecting 105 a first group 12 of individuals from a first database DB1. The first database DB1 comprises, for each of a plurality of individuals, values of the first body parameter BP1 and the first subject characteristic parameter SCP1, and the selection of the first group 12 of individuals from the first database DB1 is based on the first subject characteristic parameter values for the selected individuals being similar to the first value 12 of the first subject characteristic parameter for the subject individual 1.

Further, the method 100 comprises a step of selecting 106 a second group 22 of individuals from a second database DB2. The second database DB2 comprises, for each of the plurality of individuals, values of the body parameter BP1 and the second subject characteristic parameter SCP1, and the selection of the second group 22 of individuals from the second database DB2 is based on the second subject characteristic parameter values of the selected individuals being similar to the second value of the second subject characteristic parameter for the subject individual 1. The first and second database DB1, DB2 are in some embodiments the same database. Such common database then comprises, for each subject individual therein, values of the first body parameter BP1, the first subject characteristic parameter SCP1 and the second characteristic parameter SCP2. Different selections of individuals may be made in the two selections 105, 106 for the two groups 12, 22 of individuals. The two selections 105, 106 having different selection input of the first value of the first subject characteristic parameter SCP1 of the subject individual 1 and the second value of the second subject characteristic parameter SCP2 of the subject individual 1, respectively. When selecting the individuals for the first and/or second group 12, 22 of individuals, the selection is in some embodiments made by selecting individuals having the first and/or second subject characteristic parameter value within a predetermined interval of the first and/or second value of the first and/or second subject characteristic parameter of the subject individual. Selections may be made repeatedly until a predetermined number of individuals for the respective groups have been reached. Such predetermined number may be at least 10, at least 50, or at least 100 individuals for each group.

For the selected individuals in the two groups 12, 22 of individuals, there are provided values of the first body parameter BP1 for each individual. The method 100 comprises a step of determining 107 a first mean or median value M1 of the distribution of the first body parameter values for the individuals in the first group 12 of individuals. Further, there is a step of determining 108 a second mean or median value M2 of the distribution of the first body parameter values for the individuals in the second group 22 of individuals.

The determined first and second mean or median values M1, M2 are then used for determining 111 a first body parameter change 30, 30' by comparing the two mean or median values M1, M2, and combining with the first value 10 and the second value 20 of the first body parameter BP1 of the subject individual.

In one embodiment, the step 107 further comprises a determination of a first standard deviation SD1 of the distribution of the body parameter values for the individuals in the first group of individuals, and the step 108 further comprises a determination of a second standard deviation SD2 of the distribution of the body parameter values for the individuals in the second group of individuals. In this embodiment, the method 100 further comprises a step of determining 109 a first z-score Z1 by determining the number of first standard deviations SD1 that the first value of the first body parameter is from the first mean or median value M1, and determining 110 a second z-score Z2 by determining the number of second standard deviations SD2 that the second value of the first body parameter is from the second mean or median value M2. Finally, the two z-scores Z1, Z2 are compared to determine 111 the first body parameter change 30, 30'. The comparison may provide a difference between the two z-scores Z1, Z2. The first body parameter change 30, 30' thereby provides a basis for a weight change invariant body composition evaluation.

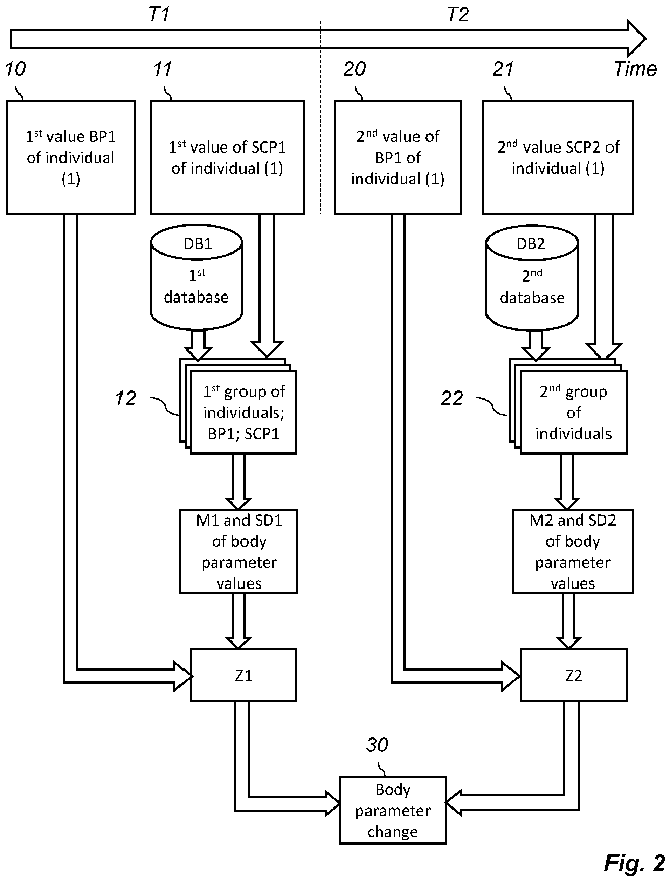

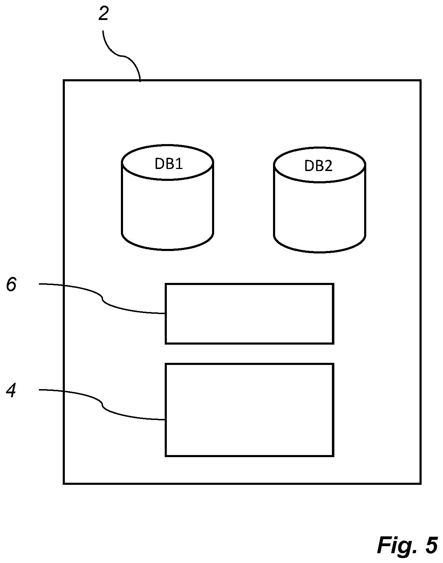

Fig. 2 further illustrates the method 100 as performed by a system 2 further described in connection with fig. 5. The first value 10 of the first body parameter BP1 of the individual 1 is acquired. The first value 10 may be received measured or determined by another entity, such as by an acquisition unit, or the system 2 may comprise an acquisition unit configured to measure or determine the first value 10. The measurement or determination may be based on an MRI scanning of the subject individual 1. The first value 11 of the first subject characteristic parameter SCP1 of the subject individual 1 is acquired. Similarly as for the first value 10, the system 2 may receive the first value 11 from the acquisition unit, or comprise the acquisition unit. The processing unit is configured to acquire the first values 10, 11. The acquisition may be directly from the acquisition unit, or from a storage unit 6 previously received the first values 10, 11. The acquisition unit and/or storage unit 6 may optionally be included in the system 2.

The first value 10 of the first body parameter BP1 and the first value 11 of the first subject characteristic parameter SCP1 relates to measurements and/or determinations of the two values 10, 11 at a first point in time T1.

The system 2 further comprises the first database DB1. The first database DB1 comprises values of the first body parameter BP1 and the first subject characteristic parameter SCP1 for each of a plurality of individuals. As discussed above, the processing unit selects a first group of individuals 12 based on the values of the first subject characteristic parameter SCP1 of each selected individual being similar to the first value 11 of the subject individual 1.

The processing unit further determines a first mean or median value M1 and a first standard deviation SD1 of the distribution of the body parameter values of the first body parameter BP1 for the individuals in the first group of individuals 12.

Further, the processing unit is configured to determine a first z-score Z1 by determining the number of first standard deviations SD1 of the first value 10 of the first body parameter BP1 from the first mean or median value M1.

At a second point in time, T2, a second value 20 of the first body parameter BP1 and a second value 21 of a second subject characteristic parameter SCP2 are measured or determined. Hence, the second values 20, 21 relates to measurements and/or determinations at a second point in time. The processing unit is configured to acquire the second values 20, 21. The acquisition may be directly from an acquisition unit, or from a storage unit previously received the second values 20, 21. The acquisition unit and/or storage unit may optionally be included in the system 2. In one embodiment, the second subject characteristic parameter SCP2 is the same parameter as the first subject characteristic parameter SCP1.

The system 2 further comprises the second database DB2. The second database DB2 comprises values of the first body parameter BP1 and the second subject characteristic parameter SCP2 for each of a plurality of individuals. As discussed above, the processing unit selects a second group of individuals 22 based on the values of the second subject characteristic parameter SCP2 of each selected individual being similar to the second value 21 of the subject individual 1. Preferably the first database DB1 and the second database DB2 is one and the same database.

The processing unit further determines a second mean or median value M2 and a second standard deviation SD2 of the distribution of the body parameter values of the first body parameter BP1 for the individuals in the second group of individuals 22.

Further, the processing unit is configured to determine a second z-score Z2 by determining the number of second standard deviations SD2 of the second value 20 of the first body parameter BP1 from the second mean or median value M2.

The first values 10, 11 relates to the first point in time T1 being different from the second point in time T2 to which the second values 20, 21 relates. The two points in time T1, T2 preferably differs in time by at least six month, or at least 12 months.

The acquisitions of the first values 10, 11 and the second values 20, 21 may be made at separate points in time than the first and second points in time T1, T2. For instance, the first values 10, 11 and the second values 20, 21 may be acquired by the processing unit at the same point in time, being a time after the second point in time T2. The first values 10, 11 and the second values 20, 21 may be stored in the storage unit and acquired therefrom by the processing unit.

The processing unit is further configured to determine the body parameter change 30 by comparing the two z-values Z1, Z2, preferably by determining a difference between Z1 and Z2.

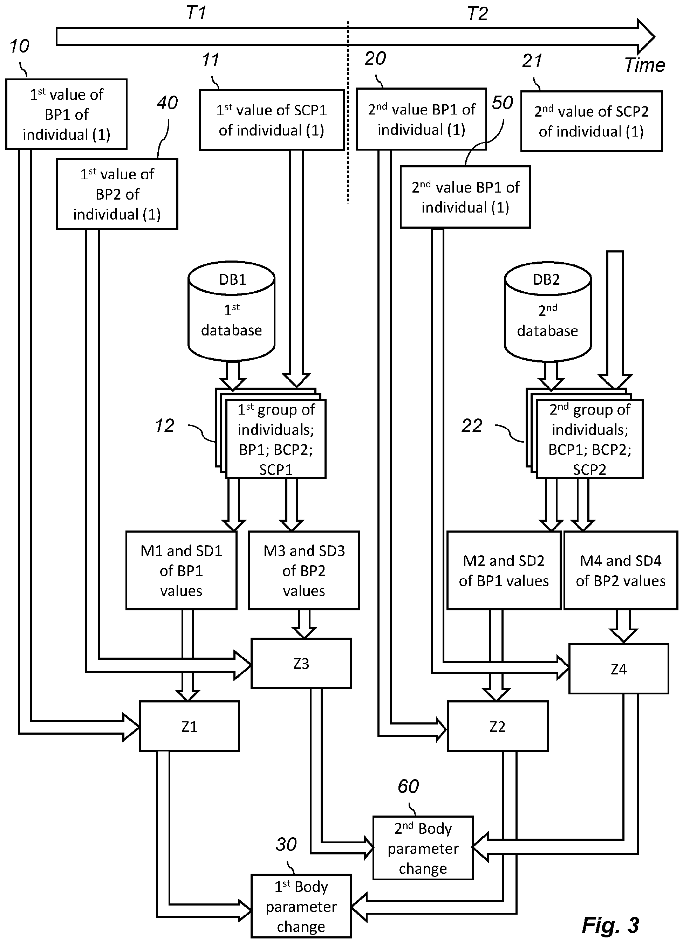

Fig. 3 illustrates an embodiment wherein a second body parameter BP2 is used as additional input for the determination of the basis for the weight invariant body composition evaluation of the subject individual 1. Such method and system comprises the same components as described above for the first embodiment. Additionally, the processing unit acquires a first value 40 of a second body parameter BP2 for the first point in time T1, and a second value of the second body parameter BP2 for the second point in time T2. Further, the first and second databases DB1, DB2, which may be one and the same database, comprise, for each individual therein, a value of the second body parameter BP2. Thereby, a third mean or median value M3 and a fourth mean or median value M4 are determined for the values of the second body parameter BP2 of the first and second groups of individuals respectively. Also, a third standard deviation SD3 and a fourth standard deviation SD4 are determined for the distribution of the values of the values of the second body parameter BP2 of the first and second groups of individuals respectively. Based on M3 and SD3, a third z-score Z3 is determined. Based on M4 and SD4, a fourth z-score Z4 is determined. The difference between Z3 and Z4 provides a second body parameter change 60. The second body parameter change may thereby be combined with the first parameter change 30 to constitute the basis for the body composition evaluation.

As an example, the first body parameter BP1 may be amount of visceral adipose tissue, and the second body parameter BP2 may be amount of liver fat. The combination of the body parameter changes 30, 60 for these two body parameters BP1, BP2 may thereby provide a more complex view of the body composition of the subject individual 1.

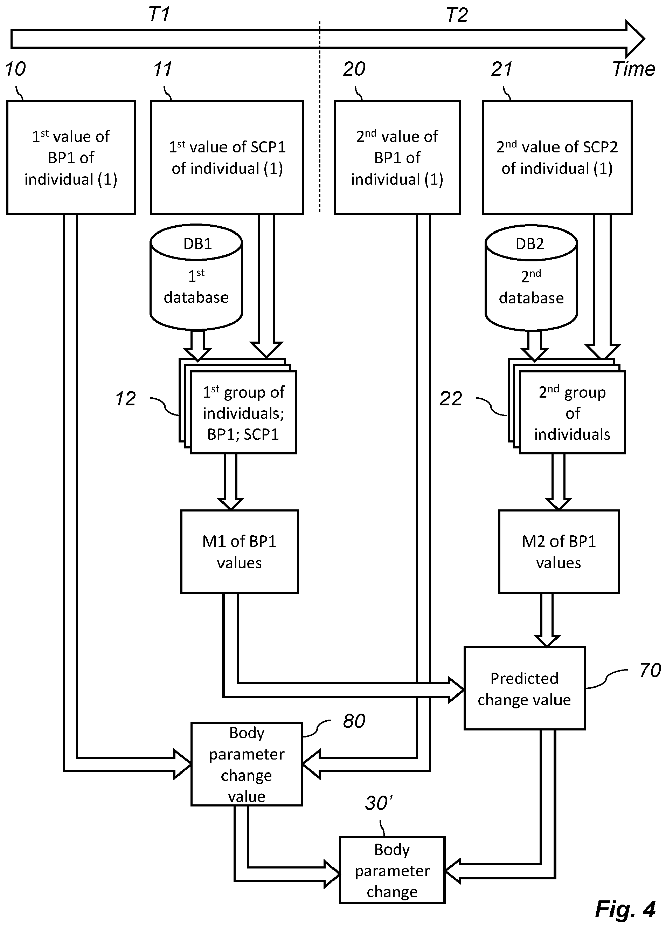

Fig. 4 illustrates a method which may be performed by the system 2 according to an alternative embodiment compared to figs. 2-3. Instead of determining z-scores Z1-Z4, the mean or median values M1, M2 are used to determine a predicted change value 70. Further, the first and second values 10, 20 of the first body parameter BP1 are used to determine a body parameter change value 80. The predicted change value 70 and the body parameter change value 80 are then compared to provide the body parameter change 30', which may serve as basis for the weight change invariant body composition evaluation of the subject individual 1. In this embodiment, the standard deviations SD1, SD2 are not necessary to determine. Instead of basing the body parameter change 30 on a comparison of z-scores, the body parameter change 30' is determined by comparing a change in values 10, 20 of the body parameter with a change in mean or median value M1, M2 from the reference groups. Hence, the deviation between the predicted body parameter change 70 from the mean or median value M1, M2 for the reference groups, and the actual body parameter change for the subject individual is determined. A deviation in an absolute value from the predicted change value 70 based on the reference groups is thereby provided. As an example, the subject individual may be determined to have lost e.g. 2 liters more visceral adipose tissue than the reference groups.

Fig. 5 illustrates a system 2 according to an embodiment of the invention. The system 2 comprises a processing unit 4 configured to perform steps of the method as disclosed herein. Further, the system 2 comprises the first database DB1 and the second database DB2. In the illustrated embodiment, the system 2 comprises a storage unit 6 configured to store the values 10, 11, 20, 21, 40, 50 of the first body parameter BP1, the second body parameter BP2, the first subject characteristic parameter SCP1 and/or the second subject characteristic parameter SCP2. The processing unit 4 may thereby acquire one or more of said values 10, 11, 20, 21, 40, 50 from the storage unit 6.

In an alternative embodiment, the system 2 may not comprise a storage unit 6. Instead, the processing unit 4 may acquire one or more of said values 10, 11, 20, 21, 40, 50 from an external source. In one embodiment, the system 2 may comprise an acquisition unit (not shown) via which the processing unit 4 may acquire the values 10, 11, 20, 21, 40, 50 from said external source. The embodiments of a storage unit 6 and an external source may be combined such that a part of the values is acquired from the storage unit 6 and a part of the values from an external source.

The processing unit 4 is configured to output the body parameter change 30, 30', 60 to be used as basis for the weight change invariant body composition evaluation.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. Method (100) of providing a basis for a weight change invariant body composition evaluation for a subject individual (1), the method being performed by a processing unit and comprising the steps of:
acquiring (101) a first value (10) of a body parameter (BP1) of the subject individual (1) for a first point in time (T1), the body parameter (BP1) being a fat or muscle related parameter;
acquiring (102) a second value (20) of the body parameter (BP1) of the subject individual (1) for a second point in time (T2) being different from the first point in time (T1);
acquiring (103) a first value (11) of a first subject characteristic parameter (SCP1) of the subject individual for the first point in time (T1), the first subject characteristic parameter (SCP1) comprising weight optionally in combination with one or more of height, age, sex, ethnicity, BMI or other body measurement;
acquiring (104) a second value (21) of a second subject characteristic parameter (SCP2) of the subject individual for the second point in time (T2), the second subject characteristic parameter (SCP2) comprising weight optionally in combination with one or more of height, age, sex, ethnicity, BMI and other body measurement;
selecting (105) a first group (12) of individuals from a first database (DB1), wherein the first database comprises, for each of a plurality of individuals, values of the body parameter (BP1) and the first subject characteristic parameter (SCP1), wherein the selection (105) of the first group (12) of individuals from the first database (DB1) is based on the first subject characteristic parameter values for the selected individuals being similar to the first value of the first subject characteristic parameter for the subject individual;
selecting (106) a second group (22) of individuals from a second database (DB2), wherein the second database comprises, for each of the plurality of individuals, values of the body parameter (BP1) and the second subject characteristic parameter (SCP2), wherein the selection (106) of the second group of individuals from the second database is based on the second subject characteristic parameter values of the selected individuals being similar to the second value of the second subject characteristic parameter for the subject individual;
determining (107) a first mean or median value (M1) of the distribution of the body parameter values for the individuals in the first group of individuals;
determining (108) a second mean or median value (M2) of the distribution of the body parameter values for the individuals in the second group of individuals;
determining (111) a first body parameter change (30, 30') by comparing the first mean or median value (M1), optionally in combination with a first standard deviation (SD1) of the distribution of the body parameter values for the individuals in the first group of individuals, with the second mean or median value (M2), optionally in combination with a second standard deviation (SD2) of the distribution of the body parameter values for the individuals in the second group of individuals, in combination with the first value (10) and the second value (20) of the body parameter (BP1) of the subject individual.

2. The method according to claim 1, further comprising the steps of
determining a first standard deviation (SD1) of the distribution of the body parameter values for the individuals in the first group of individuals, and a second standard deviation (SD2) of the distribution of the body parameter values for the individuals in the second group of individuals;
determining (109) a first z-score (Z1) by determining the number of first standard deviations (SD1) of the first value (10) of the body parameter (BP1) from the first mean or median value (M1);
determining (110) a second z-score (Z2) by determining the number of second standard deviations (SD2) of the second value (20) of the body parameter (BP1) from the second mean or median value (M2); and
wherein the step of determining (111) a first body parameter change (30) is performed by comparing the first z-score (Z1) and the second z-score (Z2).

3. The method according to any one of claims 1 and 2, further comprising the steps of
determining a predicted change value (70) by comparing the first mean or median value (M1) with the second mean or median value (M2);
determining a body parameter change value (80) by comparing the first value (10) of the body parameter (BP1) with the second value (20) of the body parameter (BP1); and
wherein the step of determining (111) the body parameter change (30') is performed by comparing the predicted change value (70) and the body parameter change value (80).

4. The method according to any one of the preceding claims, wherein the body parameter (BP1) is a parameter out of the group of amount of visceral adipose tissue, amount of abdominal subcutaneous adipose tissue, amount of liver fat, muscle volume, muscle fat infiltration, organ volume, and organ fat infiltration.

5. The method according to any one of the preceding claims, wherein the first and second points in time (T1, T2) are separated in time by at least one week, at least 1 month, at least 6 months, about 10-14 months, at least 12 months.

6. The method according to any one of the preceding claims, wherein the first subject characteristic parameter (SCP1) and the second subject characteristic parameter (SCP2) are the same subject characteristic parameter.

7. The method according to any one of the preceding claims, wherein the first subject characteristic parameter (SCP1) and/or the second subject characteristic parameter (SCP2) is weight in combination with sex.

8. The method according to any one of the claims 1-5, wherein the first subject characteristic parameter (SCP1) and/or the second subject characteristic parameter (SCP2) is weight in combination with age.

9. The method according to any one of the preceding claims, wherein the selection (105, 106) of the first and/or second group (12, 22) of individuals is made by selecting individuals from the first and/or second database (DB1, DB2) having the first and/or second subject characteristic parameter value within a predetermined interval of the first and/or second value (11, 21) of the first and/or second subject characteristic parameter (SCP1, SCP2) of the subject individual (1).

10. The method according to any one of the preceding claims, wherein the first database (DB1) and the second database (DB2) is the same database comprising, for each of the plurality of individuals therein, values of the body parameter (BP1), the first subject characteristic parameter (SCP1), and the second subject characteristic parameter (SCP2).

11. The method according to any one of the claims 2-10, wherein the step of determining (111) a body parameter change (30, 30') comprises determining a difference between the first z-score (Z1) and the second z-score (Z2) or a difference between the predicted change value (70) and the body parameter change value (80).

12. The method according to any one of the preceding claims, wherein the first and second values (10, 20) of the body parameter (BP1) of the subject individual (1) is acquired based on MRI.

13. The method according to any one of the preceding claims, wherein the first group (12) and/or the second group (22) of individuals comprises at least 10, at least 50 or at least 100 individuals selected from the first database (DB1) and/or the second database (DB2).

14. The method according to claim 2 or any one of claims 4-13 when dependent on claim 2, wherein the body parameter (BP1) is a first body parameter, and the method further comprising the steps of
acquiring a first value (40) of a second body parameter (BP2) of the subject individual (1) for the first point in time (T1), the second body parameter (BP2) being a fat or muscle related parameter different from the first body parameter (BP1);
acquiring a second value (50) of the second body parameter (BP2) of the subject individual (1) for the second point in time (T2);
wherein the first database (DB1) further comprises, for each of the plurality of individuals therein, values of the second body parameter (BP2);
wherein the second database (DB2) further comprises, for each of the plurality of individuals therein, values of the second body parameter BP2);
determining a third mean or median value (M3) and a third standard deviation (SD3) of the distribution of the second body parameter (BP2) for the individuals in the first group (10) of individuals;
determining a fourth mean or median value (M4) and a fourth standard deviation (SD4) of the distribution of the second body parameter (BP2) for the individuals in the second group (22) of individuals;
determining a third z-score (Z3) by determining the number of third standard deviations (SD3) of the first value (40) of the second body parameter (BP2) from the third mean or median value (M3);
determining a fourth z-score (Z4) by determining the number of fourth standard deviations (SD4) of the second value (50) of the second body parameter (BP2) from the fourth mean or median value (M4);
determining a second body parameter change (60) by comparing the third z-score (Z3) and the fourth z-score (Z4); and
combining the first body parameter change (30) and the second body parameter change (60).

15. A system (2) for providing a basis for an evaluation of a body parameter distribution weight change for a subject individual, the system comprising a processing unit configured to:
acquire a first value (10) of a body parameter (BP1) of the subject individual (1) for a first point in time (T1), the body parameter (BP1) being a fat or muscle related parameter;
acquire a second value (20) of the body parameter (BP1) of the subject individual (1) for a second point in time (T2) being different from the first point in time (T1);
acquire a first value (11) of a first subject characteristic parameter (SCP1) of the subject individual for the first point in time (T1), the first subject characteristic parameter (SCP1) comprising weight optionally in combination with one or more of height, age, sex, ethnicity, BMI or other body measurement;
acquire a second value (21) of a second subject characteristic parameter (SCP2) of the subject individual for the second point in time (T2), the second subject characteristic parameter (SCP2) comprising weight optionally in combination with one or more of height, age, sex, ethnicity and BMI;
select a first group (12) of individuals from a first database DB1), wherein the first database (DB1) comprises, for each of a plurality of individuals, values of the body parameter (BP1) and the first subject characteristic parameter (SCP1), wherein the selection of the first group (12) of individuals from the first database (DB1) is based on the first subject characteristic parameter values for the selected individuals being similar to the first value of the first subject characteristic parameter for the subject individual;
select a second group (22) of individuals from a second database (DB2), wherein the second database (DB2) comprises, for each of the plurality of individuals, values of the body parameter (BP1) and the second subject characteristic parameter (SCP2), wherein the selection of the second group (22) of individuals from the second database (DB2) is based on the second subject characteristic parameter values of the selected individuals being similar to the second value of the second subject characteristic parameter for the subject individual;
determine a first mean or median value (M1) of the distribution of the body parameter values for the individuals in the first group (12) of individuals;
determine a second mean or median value (M2) of the distribution of the body parameter values for the individuals in the second group (22) of individuals;
determine (111) a first body parameter change (30, 30') by comparing the first mean or median value (M1), optionally in combination with a first standard deviation (SD1) of the distribution of the body parameter values for the individuals in the first group of individuals, with the second mean or median value (M2), optionally in combination with a second standard deviation (SD2) of the distribution of the body parameter values for the individuals in the second group of individuals, in combination with the first value (10) and the second value (20) of the body parameter (BP1) of the subject individual.
